# EUROPÄISCHE PATENTSCHRIFT

(11) **EP 1 878 462 B1**
(45) Veröffentlichungstag und Bekanntmachung des Hinweises auf die Patenterteilung: **30.11.2011**
(21) Anmeldenummer: 07011929.2
(22) Anmeldetag: 19.06.2007
(51) Int. Cl.: A61M 25/06, A61N 1/05

(54) **Einführvorrichtung**
Insertion device
Dispositif d'introduction

(30) Priorität: 13.07.2006 DE 102006032583
(43) Veröffentlichungstag der Anmeldung: 16.01.2008
(73) Patentinhaber: Biotronik CRM Patent AG, 6341 Baar (CH)
(72) Erfinder: Geistert, Wolfgang, Dr., 79618 Rheinfelden (DE); Flach, Erhard, 12305 Berlin (DE)
(74) Vertreter: Lindner-Vogt, Karin L.

(56) Entgegenhaltungen:
- EP-A1- 1 151 726
- WO-A-99/33509
- GB-A- 2 268 071
- US-A- 5 029 585
- US-A1- 2003 083 560

## Beschreibung

Die Erfindung betrifft eine Einführvorrichtung zum Einführen eines medizinischen Instruments, einer Elektrodenleitung, eines Führungsdrahtes oder eines medizinischen Therapeutikums in die Körperhöhlung eines menschlichen oder tierischen Körpers gemäß dem Oberbegriff des Anspruchs 1.

Solche Einführvorrichtungen sind beispielsweise als Einführkatheter bekannt und werden zum Platzieren einer kardialen Elektrodenleitung oder eines Führungsdrahtes im Inneren des Herzen, in einem Kranzgefäß des Herzens - zum Beispiel dem Sinus Coronarius - oder in einem mit dem Herzen verbundenen anschließenden Blutgefäß - zum Beispiel der Vena Cava - verwendet. Kardiale Elektrodenleitungen sind Leitungen, die einen elektrischen Kontakt zwischen einem elektrisch aktiven Implantat wie einem Herzschrittmacher oder einem implantierbaren Kardioverter/Defibrillator (ICD) und einem elektrisch mit dem Herzen verbundenen Elektrodenkopf herstellen, um das Herz zu stimulieren oder physiologische Messwerte aufzunehmen. Besondere Ausführungen solcher Elektroden, so genannte "over the wire"-Elektroden, können mit Hilfe eines Führungsdrahtes platziert werden.

Eine Einführvorrichtung der genannten Art weist ein distales Ende, welches dem Herzen zugewandt ist, und ein proximales Ende auf, welches sich außerhalb des Körpers befindet und welches vom Operateur bedient wird. Zwischen den beiden Enden befindet sich ein aus einem flexiblen Kunststoff gefertigter Lumenabschnitt, durch dessen Lumen sowie die Öffnungen am proximalen und distalen Ende die kardiale Elektrodenleitung oder der Führungsdraht eingeführt wird. Dieser Lumenabschnitt hat einen an die Dicke der Elektrodenleitung bzw. des Führungsdrahtes angepassten Durchmesser. Nach dem Einführen der Einführvorrichtung wird - wenn das distale Ende der Einführvorrichtung an die gewünschte Stelle manövriert wurde - vom proximalen Ende aus eine Elektrodenleitung bzw. ein Führungsdraht eingeschoben und dann durch geeignete Befestigungsmittel am oder im Herzen verankert.

Ein bekanntes Problem solcher Einführvorrichtungen ist, dass ihr Entfernen nicht durch einfaches Abziehen möglich ist, da der Stecker am proximalen Ende der Elektrodenleitung wegen genormter Größe einen größeren Durchmesser als der Lumenabschnittes eines Einführkatheters aufweist. Somit ist ein Entfernen durch Schieben der Einführvorrichtung in proximale Richtung nicht möglich. Dieses Problem wird gemäß dem Stand der Technik so gelöst, dass die genannten Einführvorrichtungen vom proximalen zum distalen Ende schlitz- oder aufreißbar sind. So wird ermöglicht, dass die Einführvorrichtung nach Platzierung der Elektrodenleitung einfach entfernt werden kann.

Oft sind diese Einführvorrichtungen steuerbar ausgelegt, so dass die Platzierung vereinfacht wird.

In EP 0 898 481, US 6,159,198 und WO 99/33509 sind solche steuer- und/oder schlitzbaren Einführvorrichtungen beschrieben.

In der täglichen Praxis mit solchen Einführvorrichtungen hat es sich als wünschenswert erwiesen, während des Einführprozesses kardialer Elektroden elektrische Signale wie elektrische Potentiale oder physiologische Signale zu messen, um so einen ersten Eindruck des physiologischen Zustandes des Befestigungs-/Stimulationsortes zu erhalten. Manchmal wird es auch notwendig, Stimulationspulse abzugeben, um so den optimalen Befestigungs-/Stimulationsort der Elektrode zu finden. Dieses Problem wurde in Einführvorrichtungen aus dem Stand der Technik dadurch gelöst, dass im distalen Bereich mindestens eine Elektrode vorgesehen wurde, die die genannten Funktionen ausführen kann. Die Mess- oder Stimulationsimpulse werden beispielsweise durch externe Reizschwellenanalysatoren erzeugt. Diese Geräte können Messwerte aus dem Herzen aufnehmen und/oder verarbeiten und analysieren. Solche Geräte dienen auch weiterhin zur Abgabe von Stimulationsimpulsen und im Notfall auch zur Abgabe von Defibrillationsschocks.

WO 02/058785 oder GB 2 268 071 A zeigen eine Einführvorrichtung mit mindestens einer Elektrode im Lumenabschnitt. Die mindestens eine Elektrode ist aus einem metallischen leitenden Material gefertigt. In US 6,892,087 (veröffentlicht als US 2003/0083560) ist eine schlitz- oder aufreißbare Einführvorrichtung beschrieben, bei der eine Elektrode auf einem Dilator und die Gegenelektrode am distalen Ende der Einführvorrichtung angeordnet ist. Die mindestens eine Elektrode ist aus Metall wie z.B. Platin oder Iridium oder Legierungen daraus gefertigt.

Diese beiden genannten Einführvorrichtungen aus dem Stand der Technik haben jedoch den Nachteil, dass das Schlitzen bzw. das Aufreißen nicht oder nur sehr erschwert möglich ist. Die Elektroden müssen außerdem dünnwandig sein, um das Verhältnis Außendurchmesser zu Lumendurchmesser des Lumenabschnittes klein zu halten. Eine solche offene, dünnwandige Metallelektrode ist sehr scharfkantig und kann damit Verletzungen beim Gebrauch hervorrufen.

Daher liegt der Erfindung die Aufgabe zugrunde, die soeben genannten Nachteile des Standes der Technik zu vermeiden und eine Einführvorrichtung der im Oberbegriff des Anspruchs 1 genannten Art so zu verbessern, dass während des Gebrauchs am Befestigungs-/Stimulationsort einer Elektrodenleitung elektrische und physiologische Körpersignale gemessen werden können beziehungsweise eine Stimulation des Körpergewebes erfolgen kann, und die Elektrode weiterhin durchgängig schlitzbar bleibt.

Die Aufgabe wird erfindungsgemäß durch die Merkmale des Anspruchs 1 gelöst.

Die Vorrichtung gemäß der Erfindung beinhaltet eine Einführvorrichtung, durch welche ein medizinisches Instrument oder eine Elektrodenleitung oder ein Führungsdraht oder ein medizinisches Therapeutikum in eine Körperhöhlung eines menschlichen oder tierischen Körpers eingeführt werden kann. Es umfasst einen aus einem flexiblen Kunststoffmaterial gefertigten Lumenabschnitt mit einer Längsachse, einem proximalen und einem distalen Ende und einem das distale Ende umgebenden distalen Endbereich, mindestens ein teilkreisförmig radial um die Längsachse des Lumenabschnittes angeordnetes elektrisch leitfähiges Mittel im distalen Endbereich, um physiologische Signale abzufühlen oder das umgebende Körpergewebe geeignet zu stimulieren, sowie mindestens einen Leiter, der sich vom proximalen zum distalen Ende erstreckt und der geeignet ist, physiologische Signale zum proximalen und/oder Stimulationsimpulse zum distalen Ende zu leiten. Das mindestens eine elektrisch leitfähige Mittel ist aus einem flexiblen, nicht ausschließlich metallischen elektrisch leitfähigen Substrat hergestellt, um Traumata beim Einführen der Einführvorrichtung in den menschlichen oder tierischen Körper zu verhindern. Die Erfindung zeichnet sich dadurch aus, dass dass der Lumenabschnitt einschließlich dem elektrisch leitfähigen Mittel durch Sollbruchstellen schlitzbar oder aufreißbar ist.

Ein flexibles, nicht ausschließlich metallisches elektrisch leitfähiges Mittel kann ein leitfähiger Kunststoff (eng. "conductive plastics"), leitfähiger Klebstoff oder eine aus der Schaltkreistechnik bekannte flexible Wärmebrücke sein. Es besteht beispielsweise aus einem nicht degradablen biokompatiblen Polymer wie Polyurethan oder Pebax, in das feinste Partikel eines leitfähigen Stoffes eingebettet sind. Der eingebettete leitfähige Stoff kann beispielsweise ein Metallpulver sein, wobei als Metallpulver jedes elektrisch leitfähige Metall, vorzugsweise Au-, Ag- oder Pt-Pulver, geeignet ist. Auch Pulver aus Metalllegierungen, wie Platin/Iridium, sind geeignet. Anstelle eines Metall- oder Metalllegierungspulver kann auch Pulver aus Kohlenstoff, vorzugsweise in graphitierter Form, verwendet werden. Als besonders geeignet haben sich folgende leitfähigen Kunst- und Klebstoffe herausgestellt: Staystik, Elecolit.

Die Partikel sind so in dem Substrat eingebettet, dass sie sich zum Teil gegenseitig berühren und so einen Strompfad bilden. Der spezifische Widerstand eines solchen Substrats wird durch die Konzentration der leitfähigen Partikel beispielsweise in der Polymermatrix, durch den spezifischen Widerstand des Partikelmaterials selbst und durch die Qualität und Art der Einbettung bestimmt.
Die spezifischen Widerstände solcher leitfähiger Kunststoffe bewegen sich im Bereich von 0,05 mΩcm bis 100 Ωcm.

Der mit der Erfindung erzielte Vorteil besteht darin, dass Einführvorrichtungen mit elektrisch leitfähigen Mitteln aus einem flexiblen, nicht ausschließlich metallischen, elektrisch leitfähigen Substrat aufgrund ihrer geringeren Härte in der Anwendung im menschlichen oder tierischen Körper weniger Verletzungen hervorrufen. Ebenso sind sie leicht schlitz- oder aufreißbar.

Die elektrisch leitfähigen Mittel aus einem flexiblen, nicht ausschließlich metallischen elektrisch leitfähigen Substrat können vorgeformt hergestellt werden und dann am distalen Ende der Einführvorrichtung angeklebt, angeschweißt oder angeschmolzen werden. Alternativ kann das elektrisch leitfähige Mittel am flexiblen Lumenabschnitt angebracht werden, indem ein in Lösung befindlicher leitfähiger Kunststoff (z.B. ein leitfähiger Kleber) auf dem distalen Ende des Lumenabschnittes der Einführvorrichtung aufgetragen wird. Nach Verdunsten des Lösungsmittels bleibt das elektrisch leitfähige Mittel zurück. Auf diese Weise ist es möglich, Einführvorrichtungen mit einem elektrisch leitfähigen Mittel aus einem flexiblen, nicht ausschließlich metallischen elektrisch leitfähigen Substrat in nahezu jeder beliebigen Form und Größe herzustellen.

Vorteilhaft ist das elektrisch leitfähige Mittel am distalen Ende der Einführvorrichtung platziert und die aktive Fläche des leitfähigen Mittels in Richtung des distalen Endes ausgerichtet. Eine solche Einführvorrichtung wird orthogonal an das Gewebe herangeführt und mit dem distalen Ende an das Gewebe gedrückt. Somit ergibt sich bei einem ausschließlich in distale Richtung ausgerichteten elektrisch leitfähigen Mittel die größtmögliche Kontaktfläche zum Gewebe, was sich vorteilhaft auf die Messung von Körpersignalen und die Stimulation des Körpergewebes auswirkt.

Der elektrisch leitfähige Leiter, der das elektrisch leitfähige Mittel mit dem proximalen Ende der Einführvorrichtung verbindet, ist ein mit der Wandung des Lumenabschnittes verbundener oder in dieser Wandung eingebetteter Einzeldraht. Vorzugsweise kann die Verbindung auch über eine isoliert in die Lumenwandung eingebettete Bahn aus leitfähigem Kunststoff erfolgen. Besonders bevorzugt kann auch das in der Lumenwandung vorhandene - aus einem elektrisch leitfähigen Material bestehende - Versteifungselement oder - geflecht der Einführvorrichtung als Zuleitung verwendet werden.

In einer weiteren bevorzugten Art weist die Wandung des Lumenabschnittes mindestens drei Schichten auf, die sich entlang der Längsachse des Lumenabschnittes vom distalen zum proximalen Ende erstrecken. Eine der Schichten ist aus elektrisch leitfähigem Kunststoff und bildet den oder die elektrisch leitfähigen Leiter. Die Schicht aus elektrisch leitfähigem Kunststoff wird an deren Innen- und Außenfläche von einer dielektrischen Schicht umgeben, um eine Isolation zu bewirken.
Bevorzugt ist im distalen Endbereich mindestens eine der dielektrischen Schichten entfernt, um so eine einfache Verbindung mit einem elektrisch leitfähigen Mittel herzustellen oder dieser Bereich bildet selbst das teilkreisförmig radial um die Längsachse angeordnete elektrisch leitfähige Mittel

Natürlich ergeben sich weitere Anwendungsmöglichkeiten einer solchen Einführvorrichtung zum Beispiel bei der Platzierung von Elektrodenleitungen außerhalb des Herzens wie Leitungen zur Nervenstimulation, Leitungen zur Stimulation bestimmter Gehirnregionen oder anderen intrakorporalen Anwendungsbereichen elektrischer Therapiesignale, die von einem elektrisch aktiven Implantat ausgesendet werden. Der Gebrauch eines solchen Einführkatheters erfolgt ähnlich wie bei der Anwendung desselben am Herzen. Weitere vorteilhafte Ausgestaltungen der Erfindung sind in den abhängigen Ansprüchen beschrieben.

Es zeigen:
- Figur 1a und 1b:: das distale Ende der erfindungsgemäßen Einführvorrichtung in einer bevorzugten Ausführungsform in der Draufsicht vom distalen Ende und in der Seitenansicht;
- Figur 2a und 2b:: das distale Ende der erfindungsgemäßen Einführvorrichtung in einer weiteren bevorzugten Ausführungsform in der Draufsicht vom dista- len Ende und in der Seitenansicht;
- Figur 3a und 3b:: das distale Ende der erfindungsgemäßen Einführvorrichtung in einer anderen bevorzugten Ausführungsform in der Draufsicht vom dista- len Ende und in der Seitenansicht
- Figur 4a und 4b:: das distale Ende der erfindungsgemäßen Einführvorrichtung in einer anderen besonders bevorzugten Ausführungsform in der Draufsicht vom distalen Ende und in der Seitenansicht

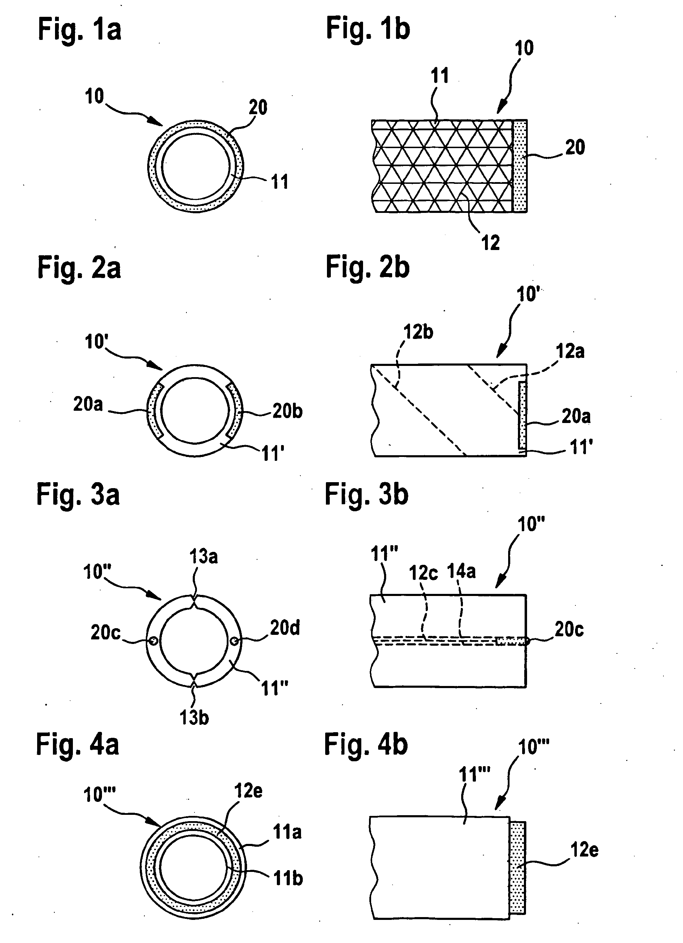

Figur 1a und 1b zeigen eine schematische Darstellung des distalen Endes eines Katheters 10, bei dem eine Elektrode 20 aus leitfähigem Kunststoff auf dem distalen Ende des Lumenabschnittes 11 angebracht ist.

Die Elektrode 20 ist mit dem metallischen Verstärkungsgeflecht 12 verbunden, welches in den Lumenabschnitt 11 eingebettet ist. Das Verstärkungsgeflecht 12 dient neben der Erhöhung der Steifigkeit des Lumenabschnittes 11 als elektrische Verbindung der Elektrode 20 mit dem nicht dargestellten, proximalen Bereich des Einführkatheters, an welchem ein Steckverbinder beliebiger, bekannter Bauart angeordnet ist. In dem Bereich, in dem die Elektrode 20 sitzt, ist der äußere Teil des Lumenabschnittes beispielsweise durch Abschleifen entfernt, so dass das Verstärkungsgeflecht 12 offengelegt wird. Die Elektrode 20 ist so auf dem distalen Ende des Katheters angebracht, dass eine elektrische Verbindung zwischen Geflecht und Elektrode besteht. Die elektrische Verbindung kann beispielsweise durch Aufschmelzen einer vorgefertigten, ringförmigen Elektrode oder durch "Aufmalen" oder Aufdrucken der Elektrode aus dickflüssigem, leitfähigen Kunststoff (z.B. leitfähigem Klebstoff) erfolgen. Alternativ kann die Elektrode auch direkt bei der Herstellung des Lumenabschnittes in diesen eingearbeitet werden.

Figur 2a und Figur 2b zeigen die schematische Darstellung einer weiteren bevorzugten Ausführungsform des distalen Endes eines Katheters 10', bei dem zwei Elektroden 20a, 20b aus leitfähigem Kunststoff teilkreisförmig auf dem distalen Ende des Lumenabschnittes 11' angebracht sind.

Am distalen Ende des Katheters 10' ist je eine der Elektroden 20a oder 20b mit jeweils einem Draht 12a oder 12b verbunden. Beide Drähte 12a, 12b sind isoliert in das Material des Lumenabschnittes 11' eingebettet. Die Drähte 12a, 12b dienen als elektrische Verbindung der Elektroden 20a, 20b mit dem nicht dargestellten, proximalen Bereich des Einführkatheters an dem ein mindestens 2-poliger Steckverbinder angeordnet ist. Die Drähte 12a, 12b können spiralförmig in den Lumenabschnitt 11' eingebettet sein.

Figur 3a und Figur 3b zeigen die schematische Darstellung einer anderen bevorzugten Ausführungsform des distalen Endes eines Katheters 10", bei dem zwei Elektroden 20c, 20d aus leitfähigem Kunststoff auf dem distalen Ende des Lumenabschnittes 11" angebracht sind. Dargestellt sind in dieser Abbildung ebenfalls zwei Sollbruchstellen 13a und 13b, die entlang der Längsachse des Katheters 10" im Lumenabschnitt vom proximalen zum distalen Ende des Katheters 10" vorgesehen sind und ermöglichen, den Lumenabschnitt 11" leicht aufzureißen. Die Sollbruchstellen 13a und 13b sind so gestaltet, dass gezielte Materialveränderungen oder Materialverjüngungen des Lumenabschnittes 11" dazu führen, dass bei einer Scherbelastung entlang der Längsachse des Lumenabschnittes ein Reißen der Wandung des Lumenabschnittes 11" an diesen vordefinierten Sollbruchstellen 13a und 13b erreicht wird. Diese Materialveränderung kann beispielsweise durch Perforation erfolgen. Weitere Ausführungsformen wie beispielsweise Aufreißdrähte entlang der Sollbruchstellen 13a und 13b können vorgesehen werden.
Der Lumenabschnitt 11" des Katheter 10" weist zwei Lumen 14a und 14b (nicht dargestellt) auf, die sich entlang der Längsachse des Katheters 10" vom distalen zum proximalen Ende erstrecken. in denen je ein Zuleitungsdraht 12c und 12d (nicht dargestellt) angeordnet sind. In der Figur 3b ist ein solches Lumen 14a mit einem Zuleitungsdraht 12c angedeutet. Der Zuleitungsdraht 12c endet kurz vor oder in etwa an dem distalen Ende des Katheters 10" und ist dort durch einen elektrischen Kontakt in die Elektrode 20c eingebettet. Die Elektrode 20c ragt auf der einen Seite ein wenig in das Lumen 14a hinein, um den elektrischen Kontakt mit dem Zuleitungsdraht 12c herzustellen und überragt auf der anderen Seite das distale Ende des Katheters 10". Auf diese Weise wird bei der Anwendung des Katheters 10" ein guter elektrischer Kontakt zwischen Elektrode 20c oder 20d und dem umgebenden Gewebe hergestellt.

Figur 4a und Figur 4b zeigen die schematische Darstellung einer anderen besonders bevorzugten Ausführungsform des distalen Endes eines Katheters 10"', bei dem die Wandung des flexiblen Lumenabschnittes 11 aus drei Schichten aufgebaut ist, die sich entlang der Längsachse des Lumenabschnittes vom proximalen zum distalen Ende erstrecken. Zwei dielektrische Schichten 11a und 11b umgeben eine elektrisch leitfähige Schicht 12e, welche als elektrische Verbindung zwischen einem elektrisch leitfähigen Mittel der vorher genannten Art und einer Steckverbindung am proximalen Ende (nicht dargestellt) dient. Die elektrisch leitfähige Schicht besteht bevorzugt aus elektrisch leitfähigem Kunststoff.
Die elektrisch leitfähige Schicht 12e kann am distalen Ende freigelegt sein. Dieses freigelegte Ende wird dadurch hergestellt, dass die dielektrischen Schichten 11a und 11b über einen Abschnitt am distalen Ende entfernt werden. Diese Entfernung kann beispielsweise durch Abschleifen, Ätzen oder andere geeignete Verfahren erfolgen. Bevorzugt kann auch nur die äußere der beiden dielektrischen Schichten 11a entfernt sein und ein elektrisch leitfähiges Mittel - wie beispielsweise in den Figuren 1a, 1b, 2a, 2b, 3a oder 3b - elektrisch verbunden werden. Die elektrische Verbindung kann beispielsweise durch Aufschmelzen einer vorgefertigten, ringförmigen Elektrode oder durch "Aufmalen" oder Aufdrucken einer Elektrode aus dickflüssigem, leitfähigem Kunststoff (z.B. leitfähigem Klebstoff) erfolgen.

## Patentansprüche

1. Einführvorrichtung (10, 10', 10"), durch welches ein medizinisches Instrument oder eine Elektrodenleitung oder ein Führungsdraht oder ein medizinisches Therapeutikum in eine Körperhöhlung eines menschlichen oder tierischen Körpers eingeführt werden kann, umfassend:
- Einen aus einem flexiblen Kunststoffmaterial gefertigten Lumenabschnitt (11, 11', 11") mit einer Längsachse, einem proximalen und einem distalen Ende und einem das distale Ende umgebenden distalen Endbereich,
- Mindestens ein teilkreisförmig radial um die Längsachse des Lumenabschnittes (11') angeordnetes elektrisch leitfähiges Mittel (20, 20a, 20b, 20c, 20d) im distalen Endbereich, um physiologische Signale abzufühlen oder das umgebende Körpergewebe geeignet zu stimulieren, wobei das mindestens eine elektrisch leitfähige Mittel (20, 20a, 20b, 20c, 20d) aus einem flexiblen, nicht ausschließlich metallischen elektrisch leitfähigen Substrat hergestellt ist, um Traumata beim Einführen der Einführvorrichtung in den menschlichen oder tierischen Körper zu verhindern, sowie
- Mindestens einen elektrisch leitfähigen Leiter (12, 12a, 12b, 12c, 12d), der sich vom proximalen zum distalen Ende erstreckt und der geeignet ist, physiologische Signale zum proximalen und/oder Stimulationsimpulse zum distalen Ende zu leiten
**dadurch gekennzeichnet, dass**
der Lumenabschnitt (11',) einschließlich dem elektrisch leitfähigen Mittel (20, 20a, 20b, 20c, 20d) durch Sollbruchstellen (13a, 13b) schlitzbar oder aufreißbar ist.

2. Einführvorrichtung nach Anspruch 1, **dadurch gekennzeichnet, dass** sich das mindestens eine elektrisch leitfähige Mittel (20, 20a, 20b, 20c, 20d) am distalen Ende des Lumenabschnittes (11, 11', 11") befindet.

3. Einführvorrichtung nach Anspruch 1 oder 2, **dadurch gekennzeichnet, dass** das mindestens eine elektrisch leitfähige Mittel (20, 20a, 20b, 20c, 20d) das distale Ende des Lumenabschnittes (11, 11', 11") bildet.

4. Einführvorrichtung nach einem der Ansprüche 1 bis 3, **dadurch gekennzeichnet, dass** sich das mindestens eine elektrisch leitfähige Mittel (20, 20a, 20b, 20c, 20d) distal des distalen Ende des Lumenabschnittes (11, 11', 11") befindet.

5. Einführvorrichtung nach einem der Ansprüche 1 bis 4, **dadurch gekennzeichnet, dass** das elektrisch leitfähige Mittel (20, 20a, 20b, 20c, 20d) und/oder der mindestens eine elektrisch leitfähige Leiter (12, 12a, 12b, 12c, 12d) aus elektrisch leitfähigem Kunststoff besteht.

6. Einführvorrichtung nach Anspruch 5, **dadurch gekennzeichnet, dass** der elektrisch leitfähige Kunststoff ein mit Metall- oder Kohlenstoffpartikeln gefülltes Polymer ist.

7. Einführvorrichtung nach einem der Ansprüche 1 bis 6, **dadurch gekennzeichnet, dass** der mindestens eine elektrisch leitfähige Leiter (12a, 12b, 12c, 12d) aus einem Einzeldraht geformt ist und der Einzeldraht parallel zur Längsachse oder spiralförmig um die Längsachse des Lumenabschnittes (11, 11', 11") verläuft.

8. Einführvorrichtung nach einem der vorhergehenden Ansprüche, **dadurch gekennzeichnet, dass** der mindestens eine elektrisch leitfähige Leiter (12, 12a, 12b, 12c, 12d) am proximalen Ende des Lumenabschnittes (11, 11', 11") mit einem Anschlusselement versehen ist, um das elektrisch leitende Mittel mit einem externen Gerät elektrisch zu verbinden.

9. Einführvorrichtung nach einem der vorhergehenden Ansprüche, **dadurch gekennzeichnet, dass** die Einführvorrichtung (10, 10', 10") steuerbar ausgelegt ist.

10. Einführvorrichtung nach Anspruch 1, **dadurch gekennzeichnet, dass** die Sollbruchstellen (13a, 13b) Materialveränderungen oder Materialverjüngungen, vorzugsweise Perforationen, sind.

11. Einführvorrichtung nach Anspruch 10, **dadurch gekennzeichnet, dass** entlang der Sollbruchstellen (13a, 13b) Aufreißdrähte vorgesehen sind.

## Claims

1. An insertion device (10, 10', 10") through which a medical instrument or an electrode line or a guide wire or a medical therapeutic can be inserted into a body cavity of a human or animal body, comprising:
- a lumen section (11, 11', 11") which is made from a flexible plastic material and has a longitudinal axis, a proximal and a distal end and a distal end region surrounding the distal end,
- at least one electrically conductive means (20, 20a, 20b, 20c, 20d) in the distal end region which means is arranged radially in a partially circular shape about the longitudinal axis of the lumen section (11') so as to sense physiological signals or to suitably stimulate the surrounding body tissue, wherein the at least one electrically conductive means (20, 20a, 20b, 20c, 20d) is made from a flexible, not exclusively metallic, electrically conductive substrate so as to prevent traumas during the insertion of the insertion device into the human or animal body, and
- at least one electrically conductive conductor (12, 12a, 12b, 12c, 12d) which extends from the proximal to the distal end and is suitable to conduct physiological signals to the proximal end and/or stimulation pulses to the distal end,
**characterized in that**
the lumen section (11') including the electrically conductive means (20, 20a, 20b, 20c, 20d) can be slit or torn open by predetermined breaking points (13a, 13b).

2. The insertion device according to claim 1, **characterized in that** the at least one electrically conductive means (20, 20a, 20b, 20c, 20d) is located at the distal end of the lumen section (11, 11', 11").

3. The insertion device according to claim 1 or claim 2, **characterized in that** the at least one electrically conductive means (20, 20a, 20b, 20c, 20d) forms the distal end of the lumen section (11, 11', 11").

4. The insertion device according to any one of the claims 1 to 3, **characterized in that** the at least one electrically conductive means (20, 20a, 20b, 20c, 20d) is located distal to the distal end of the lumen section (11, 11', 11").

5. The insertion device according to any one of the claims 1 to 4, **characterized in that** the electrically conductive means (20, 20a, 20b, 20c, 20d) and/or the at least one electrically conductive conductor (12, 12a, 12b, 12c, 12d) consists of electrically conductive plastic.

6. The insertion device according to claim 5, **characterized in that** the electrically conductive plastic is a polymer filled with metal particles or carbon particles.

7. The insertion device according to any one of the claims 1 to 6, **characterized in that** the at least one electrically conductive conductor (12a, 12b, 12c, 12d) is formed from a single wire and the single wire extends parallel to the longitudinal axis or spirally about the longitudinal axis of the lumen section (11, 11', 11").

8. The insertion device according to any one of the preceding claims, **characterized in that** the at least one electrically conductive conductor (12, 12a, 12b, 12c, 12d) is provided at the proximal end of the lumen section (11, 11', 11") with a connection element so as to electrically connect the electrically conductive means to an external apparatus.

9. The insertion device according to any of the preceding claims, **characterized in that** the insertion device (10, 10', 10") is configured to be controllable.

10. The insertion device according to claim 1, **characterized in that** the predetermined breaking points (13a, 13b) are material changes or material diminutions, preferably perforations.

11. The insertion device according to claim 10, **characterized in that** along the predetermined breaking points (13a, 13b), tear-open wires are provided.

## Revendications

1. Dispositif d'introduction (10, 10', 10"), à travers lequel un instrument médical ou une ligne d'électrodes ou un fil de guidage ou un remède médical peut être introduit dans une cavité corporelle d'un corps humain ou animal, comprenant :
- une partie de lumière (11, 11', 11") réalisée en une matière plastique souple, avec un axe longitudinal, une extrémité proximale et une extrémité distale, ainsi qu'une région d'extrémité distale entourant l'extrémité distale,
- au moins un moyen électriquement conducteur (20, 20a, 20b, 20c, 20d) disposé radialement, de façon partiellement circulaire, autour de l'axe longitudinal de la partie de lumière (11'), dans la région d'extrémité distale, pour détecter des signaux physiologiques ou stimuler de façon appropriée les tissus corporels environnants, l'au moins un moyen électriquement conducteur (20, 20a, 20b, 20c, 20d) étant fabriqué à partir d'un substrat électriquement conducteur non-exclusivement métallique, pour empêcher les traumatismes lors de l'introduction du dispositif d'introduction dans le corps humain ou animal, et
- au moins un conducteur électriquement conducteur (12, 12a, 12b, 12c, 12d) s'étendant de l'extrémité proximale à l'extrémité distale, adapté pour conduire des signaux physiologiques vers l'extrémité proximale et/ou des impulsions de stimulation vers l'extrémité distale,
**caractérisé en ce que**
la partie de lumière (11'), y compris le moyen électriquement conducteur (20, 20a, 20b, 20c, 20d), peut être fendue ou ouverte par une zone de rupture privilégiée (13a, 13b).

2. Dispositif d'introduction selon la revendication 1, **caractérisé en ce qu'**au moins un moyen électriquement conducteur (20, 20a, 20b, 20c, 20d) se trouve à l'extrémité distale de la partie de lumière (11, 11', 11").

3. Dispositif d'introduction selon la revendication 1 ou 2, **caractérisé en ce que** l'au moins un moyen électriquement conducteur (20, 20a, 20b, 20c, 20d) forme l'extrémité distale de la partie de lumière (11, 11', 11").

4. Dispositif d'introduction selon l'une des revendications 1 à 3, **caractérisé en ce que** l'au moins un moyen électriquement conducteur (20, 20a, 20b, 20c, 20d) est situé de façon distale par rapport à l'extrémité distale de la partie de lumière (11, 11', 11 ").

5. Dispositif d'introduction selon l'une des revendications 1 à 4, **caractérisé en ce que** le moyen électriquement conducteur (20, 20a, 20b, 20c, 20d) et/ou l'au moins un conducteur électriquement conducteur (12, 12a, 12b, 12c, 12d) sont constitués d'une matière plastique électriquement conductrice.

6. Dispositif d'introduction selon la revendication 5, **caractérisé en ce que** la matière plastique électriquement conductrice est un polymère chargé de particules métalliques ou de particules de carbone.

7. Dispositif d'introduction selon l'une des revendications 1 à 6, **caractérisé en ce que** l'au moins un conducteur électriquement conducteur (12a, 12b, 12c, 12d) est constitué d'un fil unique, et le fil unique s'étend parallèlement à l'axe longitudinal ou en forme de spirale autour de l'axe longitudinal de la partie de lumière (11, 11', 11 ").

8. Dispositif d'introduction selon l'une des revendications précédentes, **caractérisé en ce que** l'au moins un conducteur électriquement conducteur (12, 12a, 12b, 12c, 12d) est pourvu d'un élément de raccordement à l'extrémité proximale de la partie de lumière (11, 11', 11 "), pour relier le moyen électriquement conducteur à un appareil électrique externe.

9. Dispositif d'introduction selon l'une des revendications précédentes, **caractérisé en ce que** le dispositif d'introduction (10, 10', 10") est conçu de façon à pouvoir être commandé.

10. Dispositif d'introduction selon la revendication 1, **caractérisé en ce que** les zones de rupture privilégiées (13a, 13b) sont des altérations de matériau ou des affaiblissements de matériau, de préférence des perforations.

11. Dispositif d'introduction selon la revendication 10, **caractérisé en ce que** des fils d'ouverture sont prévus le long des zones de rupture privilégiées (13a, 13b).
